# EUROPEAN PATENT APPLICATION

(11) **EP 4 696 321 A1**
(43) Date of publication of application: **18.02.2026**
(21) Application number: 24788833.2
(22) Date of filing: 12.04.2024
(51) Int. Cl.: A61K 39/395, A61K 9/08, C07K 1/00, C07K 16/46, C12M 1/24, C12P 21/08

(54) **METHOD FOR STABILIZING PROTEIN-CONTAINING PHARMACEUTICAL PREPARATION**

(30) Priority: 14.04.2023 JP 2023066625
(71) Applicant: CHUGAI SEIYAKU KABUSHIKI KAISHA, Tokyo 115-8543 (JP)
(72) Inventor: ARAI, Kengo, Tokyo 115-8543 (JP); NARITA, Yusuke, Tokyo 115-8543 (JP); SOEDA, Kohei, Tokyo 115-8543 (JP); MARUYAMA, Minako, Tokyo 115-8543 (JP); EGAMI, Kiichi, Tokyo 115-8543 (JP)
(74) Representative: Vossius & Partner Patentanwälte Rechtsanwälte mbB
(86) International application number: PCT/JP2024/014819
(87) International publication number: WO 2024/214811

(57) **Abstract**

The present invention provides a method for reducing, in a pharmaceutical formulation comprising a protein as an active ingredient in a solution, adsorption of the protein to a container and formation of particles in the solution, wherein a material of the container is glass, an inside surface of the container has a coating, at least a part of the inside surface has a water contact angle of 90° or more, and the particles have a particle size of 40 µm or more.

## Description

### TECHNICAL FIELD

The present invention relates to a method for stabilizing a pharmaceutical formulation comprising a protein such as an antibody, and particularly relates to a method for improving storage stability of a liquid pharmaceutical formulation. The present invention further relates to an antibody-containing pharmaceutical formulation having improved storage stability.

### BACKGROUND ART

In recent years, various formulations comprising a protein such as an antibody as an active ingredient have been developed and put into practical use, and dosage forms of many of these are intravenous injection or subcutaneous injection. In either of the dosage forms, vial formulations are widely used because a dose can be adjusted according to a patient, and the production is easy. Actually, more than a half of biopharmaceuticals launched in Japan in 2020 are liquid formulations filled in vials (Non Patent Literature 1).

Many of vial formulations each include a glass or polymer vial filled with a drug solution, and a rubber stopper, and are provided in a state sealed with an aluminum or plastic cap. In actual medical practice, the cap is removed before use, and a needle of a syringe is punctured in a portion of the thus exposed stopper. Then, the drug solution is aspirated, and adjusted to a necessary amount, followed by administration.

Particularly in an antibody-containing formulation, formation of particles in an aqueous solution becomes a problem. The particles to be formed are aggregates derived from an antibody, and sub-visible particles (SVPs), that is, microparticles with a particle size of 1.5 µm to smaller than 50 µm that are generally difficult to see with eyes, and visible particles (VPs, larger than 100 µm) that are visually detectable under standard illuminance (about 2,000 to 3,000 lx) are known. A visual detection rate of visible particles in a pharmaceutical formulation varies greatly depending on an examiner, and under standard illuminance prescribed in the Japanese pharmacopoeia (about 2,000 to 3,000 1x), it is reported that detection sensitivity of particles with a particle size of 100 µm is about 40%, detection sensitivity of particles with a particle size of 150 µm is about 70%, and detection sensitivity of particles with a particle size of 200 µm is almost 100% (Non Patent Literature 2). Actually, particles with a smaller particle size of a minimum of about 40 µm can be visually detected by increasing illuminance for observing a pharmaceutical formulation or by increasing an observation time period.

Generally, antibodies have a property of adsorbing to and aggregating on interfaces such as air-liquid interfaces and solid-liquid interfaces. The presence of such interfaces may contribute to the formation of the visible particles (VPs) that are aggregates generally larger than SVPs. An antibody solution filled in a vial forms a gas-liquid interface owing to the presence of a head space, and forms a solid-liquid interface through contact with the vial and the stopper.

There are many reports on the formation of protein particles caused by the presence of such interfaces. It has been reported that the amount of proteins adsorbed to the surfaces of a syringe and a metal affects the amount of SVPs formed thereafter (Non Patent Literatures 3 and 4).

To many of rubber stoppers, a silicone oil is applied for the purpose of improvement of efficiency in unmolding at the time of molding, and prevention of adhesion between members. In this case, the antibody solution contacts the silicone oil to form a new solid-liquid interface. It has been reported that the antibody solution adsorbs and aggregates on such a solid-liquid interface to appear as visible particles (Non Patent Literature 4). Besides, it has been reported that when a mechanical stress is applied to a vial filled with an antibody solution, the number of microparticles remarkably increases due to the presence of the interface. In this case, the influence of the difference in the surface state (roughness) of the vial on the amount of microparticles to be formed has been studied (Non Patent Literature 6).

A method for reducing the stress applied to each of various interfaces can be improvement of the surface state inside a vial. It has been studied that adsorption and desorption of aggregates on a gas-liquid interface and a solid-liquid interface are inhibited by adjusting the surface property of a vial, and as a result, formation of SVPs and visible particles is inhibited (Patent Literatures 1 to 3).

The relation between the surface property of a solid and protein adsorption thereto has been studied. In adsorption of an antibody to borosilicate glass, it has been suggested that electrostatic interaction between an antibody molecule and glass surface, and between adsorbed antibody molecules is dominant, and a contribution of other driving power such as hydrophobic interaction and structural change caused in the surface is very small. Actually, it has been reported that the amount of adsorption of IgG1 to a cycloolefin polymer (COP) vial is larger than the amount of adsorption to a glass vial under a condition of high ionic strength (Non Patent Literature 7). On the other hand, it is regarded that electrostatic interaction is difficult to occur in COP, and hence it has been revealed that adsorption, using electrostatic interaction as driving power, to the inside wall of a syringe barrel of a protein for treatment can be inhibited by using a COP as compared with that using borosilicate glass (Non Patent Literatures 8 and 9).

Based on test results obtained using fibronectin, adsorption of a protein to a hydrophilic surface with a small contact angle has been examined (Non Patent Literature 10). Besides, adsorption of a protein to a hydrophobic surface has been also reported (Non Patent Literature 11).

There is a known vial surface-treated by a silicone coating treatment or the like for the purpose of preventing alkaline elution from a container member and adsorption of a content liquid (Patent Literature 4).

### CITATION LIST

### PATENT LITERATURE

[Patent Literature 1] WO 2019/064263A1
[Patent Literature 2] WO 2021/094508A1
[Patent Literature 3] WO 2003/103398A1
[Patent Literature 4] WO 2008/071458A1
[Patent Literature 5] Japanese Patent Laid-Open No. 2020-146466

### NON PATENT LITERATURE

[Non Patent Literature 1] Expert Opinion on Drug Delivery, Volume 18, Issue 4, Jan 2021, Pages 459-470.
[Non Patent Literature 2] James A. Melchore, AAPS PharmSciTech; 2011; 12(1): 215-221
[Non Patent Literature 3] Mol. Pharmaceutics 2020, 17, 2, 569-578.
[Non Patent Literature 4] J Pharm Sci. Volume 107, Issue 6, June 2018, Pages 1521-1529.
[Non Patent Literature 5] J Pharm Sci. 2022 Aug; 111(8):2191-2200.
[Non Patent Literature 6] J Pharm Sci. 2020 Mar; 109(3):1270-1280.
[Non Patent Literature 7] Eur J Pharm Biopharm. 2011 Jun; 78(2):239-247.
[Non Patent Literature 8] J Pharm Sci. 2021 Nov; 110(11):3568-3579.
[Non Patent Literature 9] J Pharm Sci. 2018 Jun; 107(6):1521-1529.
[Non Patent Literature 10] Angew Chem Int Ed Engl. 2014 Jul 28;53(31):8004-31.
[Non Patent Literature 11] Adv Colloid Interface Sci. 2011 Feb 17;162(1-2):87-106.

### SUMMARY OF INVENTION

### TECHNICAL PROBLEM

In general, an antibody solution has a property of adsorbing to an interface such as a gas-liquid interface or a solid-liquid interface to aggregate thereon. The presence of such an interface may make a contribution to the formation of SVPs and visible particles that are generally larger aggregates. An antibody solution filled in a glass vial contacts the inside surface of the vial to form a solid-liquid interface. Besides, when the surface of the vial or the stopper is treated with silicone, a biopharmaceutical solution contacts silicone on the solid-phase surface to form a new solid-liquid interface. It has been reported that an antibody solution adsorbs to such interfaces to aggregate, and appears as visible particles.

An object of the present invention is to provide a method for preventing, in an antibody-containing liquid pharmaceutical formulation filled in a container such as a vial, formation of visible particles to improve storage stability of the pharmaceutical formulation.

### SOLUTION TO PROBLEM

As a result of making studies to solve the above-described problems, the present inventors have found that the problems are solved by using a container having a specific property, resulting in accomplishing the present invention. In one aspect, the present invention discloses the following inventions.
[A-1] A method for reducing, in a pharmaceutical formulation comprising a protein as an active ingredient in a solution, adsorption of the protein to a container and formation of particles in the solution, wherein a material of the container is glass, an inside surface of the container has a coating, at least a part of the inside surface has a water contact angle of 90° or greater, and the particles have a particle size of 40 µm or greater.
[A-2] The method according [A-1], wherein the solution is an aqueous solution.
[A-3] The method according to [A-1] or [A-2], wherein the protein is a monoclonal antibody, a protein fusion antibody, or a peptide fusion antibody.
[A-4] The method according to [A-3], wherein the protein is a monoclonal antibody, and the monoclonal antibody is any one of a monospecific antibody, a bispecific antibody, and a multi-specific antibody capable of binding to three or more antigens.
[A-5] The method according to any one of [A-1] to [A-4], wherein the coating is a coating of a hydrophobic compound containing elements Si, C, O, and H.
[A-6] The method according to [A-5], wherein a content in the hydrophobic compound of other elements except for Si, C, O and H is lower than 10%, and the compound is represented by a composition SiOₓC_{y}H_{z}, wherein x is in a range of 0.0 to 1.2, y is in a range of 0.0 to 6.0, and z is in a range of 0.0 to 6.0.
[A-7] The method according to [A-6], wherein x is smaller than 1.
[A-8] The method according to [A-6] or [A-7], wherein x is in a range of 0.6 to 0.9, and y is in a range of 1.2 to 3.3.
[A-9] The method according to any one of [A-5] to [A-8], wherein the hydrophobic compound is a polymer.
[A-10] The method according to any one of [A-1] to [A-9], wherein the inside surface of the container has a water contact angle of 95° or greater.
[A-11] The method according to any one of [A-1] to [A-10], wherein the container is a vial, a syringe, or a cartridge.
[A-12] The method according to any one of [A-1] to [A-11], wherein the container is a vial.
[A-13] The method according to any one of [A-1] to [A-12], wherein the solution comprises one or a plurality of pharmaceutically acceptable excipients including a buffer, a preservative, an antioxidant, a chelating agent, a cryoprotective agent, a surfactant, a tonicity agent, and a stabilizing agent, or a combination thereof.
[A-14] The method according to any one of [A-1] to [A-13], wherein the solution comprises a surfactant selected from polysorbate, poloxamer, sodium lauryl sulfate, polyol, poly(ethylene glycol), glycerol, propylene glycol, and poly(vinyl alcohol).
[A-15] The method according to any one of [A-1] to [A-14], wherein the solution comprises a surfactant of poloxamer 188.
[A-16] The method according to any one of [A-13] to [A-15], wherein a concentration of the surfactant in the solution is 0.01 mg/mL or greater.
[A-17] The method according to any one of [A-13] to [A-16], wherein a concentration of the surfactant in the solution is in a range of 0.01 to 5 mg/mL.
[A-18] The method according to any one of [A-13] to [A-17], wherein a concentration of the surfactant in the solution is in a range of 0.25 to 0.75 mg/mL.
[A-19] The method according to any one of [A-1] to [A-18], wherein a pH of the solution is in a range of 4.5 to 7.5.
[A-20] The method according to any one of [A-1] to [A-19], wherein a pH of the solution is in a range of 5.0 to 7.0.
[A-21] The method according to any one of [A-1] to [A-20], wherein a pH of the solution is in a range of 5.5 to 6.5.
[A-22] The method according to any one of [A-1] to [A-21], wherein a dose of the protein is 10 µg or greater, 50 µg or greater, 100 µg or greater, 500 µg or greater, 1,000 µg or greater, 5,000 µg or greater, or 10,000 µg or greater.
[A-23] The method according to any one of [A-1] to [A-22], wherein a dose of the protein is 100 µg or greater.
[A-24] The method according to any one of [A-1] to [A-23], wherein a concentration of the protein in the solution is 0.01 mg/mL or greater.
[A-25] The method according to any one of [A-1] to [A-24], wherein a concentration of the protein in the solution is in a range of 0.01 to 1.00 mg/mL, 1.01 to 9.99 mg/mL, 10.0 to 99.9 mg/mL, or 100 to 200 mg/mL.
[A-26] The method according to any one of [A-1] to [A-25], wherein a concentration of the protein in the solution is in a range of 1 to 100 mg/mL, 2 to 80 mg/mL, 3 to 60 mg/mL, 4 to 50 mg/mL, 5 to 30 mg/mL, or 6 to 20 mg/mL.
[A-27] The method according to any one of [A-1] to [A-26], wherein the solution comprises a stabilizing agent selected from arginine, aspartic acid, methionine, and sucrose.
[A-28] The method according to any one of [A-1] to [A-27], wherein the solution comprises a stabilizing agent of arginine and aspartic acid.
[A-29] The method according to any one of [A-13], [A-27], and [A-28], wherein a concentration of the stabilizing agent in the solution is 0.1 mmol/L or greater.
[A-30] The method according to any one of [A-13], and [A-27] to [A-29], wherein a concentration of the stabilizing agent in the solution is in a range of 1 to 500 mmol/L.
[A-31] The method according to any one of [A-1] to [A-30], wherein the solution comprises a buffer selected from histidine, citric acid, acetic acid, trihydroxy methyl aminomethane, phosphoric acid, and succinic acid.
[A-32] The method according to any one of [A-1] to [A-31], wherein the solution comprises a buffer of histidine.
[A-33] The method according to any one of [A-13], [A-31], and [A-32], wherein a concentration of the buffer in the solution is 1 mmol/L or greater.
[A-34] The method according to any one of [A-13], and [A-31] to [A-33], wherein a concentration of the buffer in the solution is in a range of 5 to 100 mmol/L.
[A-35] The method according to any one of [A-1] to [A-34], wherein the protein is a monoclonal antibody selected from an antibody having an H-chain of SEQ ID NO: 1 and an L-chain of SEQ ID NO: 2, and an antibody having an H-chain of SEQ ID NO: 3 and an L-chain of SEQ ID NO: 4, and an H-chain of SEQ ID NO: 6 and SEQ ID NO: 5.
[A-36] The method according to any one of [A-1] to [A-35], wherein the coating is a coating of polydimethylsiloxane (PDMS).
[B-1] A pharmaceutical formulation comprising a protein as an active ingredient in a solution, wherein a material of a container is glass, an inside surface of the container has a coating, at least a part of the inside surface has a water contact angle of 90° or greater, and particles have a particle size of 40 µm or greater, and
   (1) a ratio of visible particles detected in the solution after static storage at 40°C for 2 months after production is 25% or smaller,
   (2) a ratio of visible particles detected in the solution after static storage at 40°C for 3 months after production is 35% or smaller, or
   (3) a ratio of visible particles detected in the solution after static storage at 40°C for 4 months after production is 45% or smaller.
[B-2] The pharmaceutical formulation according [B-1], wherein the solution is an aqueous solution.
[B-3] The pharmaceutical formulation according to [B-1] or [B-2], wherein the protein is a monoclonal antibody.
[B-4] The pharmaceutical formulation according to [B-3], wherein the monoclonal antibody is either of a monospecific antibody and a bispecific antibody.
[B-5] The pharmaceutical formulation according to any one of [B-1] to [B-4], wherein the coating is a coating of a hydrophobic compound containing elements Si, C, O, and H.
[B-6] The pharmaceutical formulation according to [B-5], wherein a content in the hydrophobic compound of other elements except for Si, C, O and H is lower than 10%, and the compound is represented by a composition SiOₓC_{y}H_{z}, wherein x is in a range of 0.0 to 1.2, y is in a range of 0.0 to 6.0, and z is in a range of 0.0 to 6.0.
[B-7] The pharmaceutical formulation according to [B-6], wherein x is smaller than 1.
[B-8] The pharmaceutical formulation according to [B-6] or [B-7], wherein x is in a range of 0.6 to 0.9, and y is in a range of 1.2 to 3.3.
[B-9] The pharmaceutical formulation according to any one of [B-5] to [B-8], wherein the hydrophobic compound is a polymer.
[B-10] The pharmaceutical formulation according to any one of [B-1] to [B-9], wherein an inside surface of the container has a water contact angle of 95° or greater.
[B-11] The pharmaceutical formulation according to any one of [B-1] to [B-10], wherein the container is a vial, a syringe, or a cartridge.
[B-12] The pharmaceutical formulation according to any one of [B-1] to [B-11], wherein the container is a vial.
[B-13] The pharmaceutical formulation according to any one of [B-1] to [B-12], wherein the solution comprises one or a plurality of pharmaceutically acceptable excipients including a buffer, a preservative, an antioxidant, a chelating agent, a cryoprotective agent, a surfactant, a tonicity agent, a stabilizing agent, or a combination thereof.
[B-14] The pharmaceutical formulation according to any one of [B-1] to [B-13], wherein the solution comprises a surfactant selected from polysorbate, poloxamer, sodium lauryl sulfate, polyol, poly(ethylene glycol), glycerol, propylene glycol, and poly(vinyl alcohol).
[B-15] The method according to any one of [B-1] to [B-14], wherein the solution comprises a surfactant of poloxamer 188.
[B-16] The pharmaceutical formulation according to any one of [B-13] to [B-15], wherein a concentration of the surfactant in the solution is 0.01 mg/mL or greater.
[B-17] The pharmaceutical formulation according to any one of [A-13] to [B-16], wherein a concentration of the surfactant in the solution is in a range of 0.01 to 5 mg/mL.
[B-18] The pharmaceutical formulation according to any one of [B-13] to [B-17], wherein a concentration of the surfactant in the solution is in a range of 0.25 to 0.75 mg/mL.
[B-19] The pharmaceutical formulation according to any one of [B-1] to [B-18], wherein a pH of the solution is in a range of 4.5 to 7.5.
[B-20] The pharmaceutical formulation according to any one of [B-1] to [B-19], wherein a pH of the solution is in a range of 5.0 to 7.0.
[B-21] The pharmaceutical formulation according to any one of [B-1] to [B-20], wherein a pH of the solution is in a range of 5.5 to 6.5.
[B-22] The pharmaceutical formulation according to any one of [B-1] to [B-21], wherein a dose of the protein is 10 µg or greater, 50 µg or greater, 100 µg or greater, 500 µg or greater, 1,000 µg or greater, 5,000 µg or greater, or 10,000 µg or greater.
[B-23] The pharmaceutical formulation according to any one of [B-1] to [B-22], wherein a dose of the protein is 500 µg or greater.
[B-24] The pharmaceutical formulation according to any one of [B-1] to [B-23], wherein a concentration of the protein in the solution is 0.01 mg/mL or greater.
[B-25] The pharmaceutical formulation according to any one of [B-1] to [B-24], wherein a concentration of the protein in the solution is in a range of 0.01 to 1.00 mg/mL, 1.01 to 9.99 mg/mL, 10.0 to 99.9 mg/mL, or 100 to 200 mg/mL.
[B-26] The pharmaceutical formulation according to any one of [B-1] to [B-25], wherein a concentration of the protein in the solution is in a range of 1 to 100 mg/mL, 2 to 80 mg/mL, 3 to 60 mg/mL, 4 to 50 mg/mL, 5 to 30 mg/mL, or 6 to 20 mg/mL.
[B-27] The pharmaceutical formulation according to any one of [B-1] to [B-26], wherein the ratio of detecting visible particles in the solution is a ratio of detecting visible particles in 10 pharmaceutical formulations consecutively produced.
[B-28] The pharmaceutical formulation according to any one of [B-1] to [B-26], wherein the ratio of detecting visible particles in the solution is a ratio of detecting visible particles in 100 pharmaceutical formulations consecutively produced.
[B-29] The pharmaceutical formulation according to any one of [B-1] to [B-26], wherein the ratio of detecting visible particles in the solution is a ratio of detecting visible particles in one lot of pharmaceutical formulations consecutively produced.
[B-30] The pharmaceutical formulation according to any one of [B-1] to [B-29], wherein the ratio of visible particles detected in the solution after static storage at 40°C for 4 months after production is 45% or smaller.
[B-31] The method according to any one of [B-1] to [B-30], wherein the solution comprises a stabilizing agent selected from arginine, aspartic acid, methionine, and sucrose.
[B-32] The pharmaceutical formulation according to any one of [B-1] to [B-31], wherein the solution comprises a stabilizing agent of arginine and aspartic acid.
[B-33] The pharmaceutical formulation according to any one of [B-13], [B-31], and [B-32], wherein a concentration of the stabilizing agent in the solution is 0.1 mmol/L or greater.
[B-34] The pharmaceutical formulation according to any one of [B-13], and [B-31] to [B-33], wherein a concentration of the stabilizing agent in the solution is in a range of 1 to 500 mmol/L.
[B-35] The pharmaceutical formulation according to any one of [B-1] to [B-34], wherein the solution comprises a buffer selected from histidine, citric acid, acetic acid, trihydroxy methyl aminomethane, phosphoric acid, and succinic acid.
[B-36] The pharmaceutical formulation according to any one of [B-1] to [B-35], wherein the solution comprises a buffer of histidine.
[B-37] The pharmaceutical formulation according to any one of [B-13], [B-35], and [B-36], wherein a concentration of the buffer in the solution is 1 mmol/L or greater.
[B-38] The pharmaceutical formulation according to any one of [B-13], and [B-35] to [B-37], wherein a concentration of the buffer in the solution is in a range of 5 to 100 mmol/L.
[B-39] The pharmaceutical formulation according to any one of [B-1] to [B-38], wherein the protein is a monoclonal antibody selected from an antibody having an H-chain of SEQ ID NO: 1 and an L-chain of SEQ ID NO: 2, and an antibody having an H-chain of SEQ ID NO: 3 and an L-chain of SEQ ID NO: 4, and an H-chain of SEQ ID NO: 6 and SEQ ID NO: 5.
[B-40] The pharmaceutical formulation according to any one of [B-1] to [B-39], wherein the ratio of visible particles detected in the solution after static storage at 40°C for 2 months after production is 25% or smaller.
[B-41] The pharmaceutical formulation according to any one of [B-1] to [B-40], wherein the coating is a coating of polydimethylsiloxane (PDMS).
[B-42] The pharmaceutical formulation according to any one of [B-1] to [B-41], for use in the method according to any one of [A-1] to [A-36].
[C-1] A pharmaceutical composition comprising a protein as an active ingredient in a solution, for use in the method according to any one of [A-1] to [A-36].

### ADVANTAGEOUS EFFECTS OF INVENTION

According to the present invention, a method for inhibiting, in an antibody-containing liquid pharmaceutical formulation filled in a container such as a vial, formation of visible particles to improve storage stability of the pharmaceutical formulation is provided. In one aspect of the present invention, an effect of inhibiting formation of visible particles can be confirmed, for example, through comparison with a case in which an uncoated glass container is used instead of a container of the present invention.

### BRIEF DESCRIPTION OF DRAWING

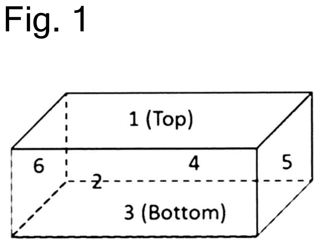

Figure 1 is a schematic diagram of a cardboard box used in a drop test, illustrating numbering of respective surfaces.

### DESCRIPTION OF EMBODIMENTS

In one aspect of the present invention, a glass vial can be used as a container, and the inside surface of the glass vial has a coating. The glass vial having a coating on the inside surface can be produced by a known method, such as methods described in Patent Literatures 4 and 5.

In one aspect of the present invention, the glass vial to be used as the container can be obtained by purchase, and for example, TopLyo(R) (SCHOTT), and silicone-coated vial (DAIWA SPECIAL GLASS Co., Ltd.) and the like can be used.

In one aspect of the present invention, a vial having a coating on the inside surface can be used, and the coating contains a compound comprising elements Si, C, O, and H, a content of other elements except for Si, C, O and H is lower than 10%, and the compound is represented by a composition SiOₓC_{y}H_{z} (wherein x is smaller than 1), and has a contact angle against water of 90° or greater.

In one aspect of the present invention, borosilicate glass having the following characteristics is used as the container: Assuming that a portion of the borosilicate glass container from the innermost surface to a depth of 10 nm, that is, a detection depth in X-ray photoelectron spectroscopy, is referred to as the inside surface, a content of SiO₂ in the inside surface is 85 to 92 wt%, one or more oxides selected from Na₂O, K₂O, CaO, BaO, B₂O₃, and Al₂O₃ are formed in addition to SiO₂ in the inside surface, aluminum and boron are fixed, in the inside surface, in a network formation having a siloxane structure in which silicon and oxygen are bonded via a siloxane bond, an alkali metal and/or an alkali earth metal is fixed in the siloxane structure through an ionic bond, there is no discontinuity between the inside surface and an inner portion of the container, and the composition is consecutively changed from the inside surface to the inner portion of the container.

In one aspect of the present invention, a container having a silicone coating on the inside surface can be used. In one embodiment, polydimethylsiloxane (PDMS; dimethylpolysiloxane, or dimethicone) can be used as the silicone coating. Specifically, the composition of the silicone is CH₃[Si(CH₃)₂O]ₙSi(CH₃)₃.

In one aspect of the present invention, a syringe, a cartridge, or the like can be used as the container, and such a container can be used as a container provided with a coating by a known method described herein above.

In one aspect of the present invention, a ratio of visible particles detected in a solution after static storage at 40°C for 2 months after production can be confirmed by actually subjecting the produced pharmaceutical to a test. In one aspect of the present invention, a ratio of visible particles detected in a solution after static storage at 40°C for 3 months after production can be confirmed by actually subjecting the produced pharmaceutical to a test. In one aspect of the present invention, a ratio of visible particles detected in a solution after static storage at 40°C for 4 months after production can be confirmed by actually subjecting the produced pharmaceutical to a test. In one embodiment, the test can be performed using a prescribed number of pharmaceuticals consecutively produced in a plant. Besides, the ratio in pharmaceuticals produced in the plant can be confirmed by confirming the ratio in pharmaceuticals produced in a laboratory by those skilled in the art with the same prescription and container used in consideration of production conditions employed in the plant. In one embodiment of the present invention, a ratio of visible particles detected in one lot of pharmaceutical formulations consecutively produced can be confirmed by, for example, testing a prescribed number (for example, 10, 1,000, or the like) of the lot of pharmaceuticals, or can be confirmed by testing pharmaceuticals produced in a laboratory under the same conditions and in the same manner as the lot, or can be confirmed by testing a prescribed number of pharmaceuticals of another lot produced under the same conditions and in the same manner as the lot.

Herein, a test for statically storing pharmaceuticals at 40°C can be performed, for example, by a test method described in The Japanese Pharmacopoeia. In one aspect of the present invention, the formation of visible particles in a pharmaceutical formulation can be confirmed by a known test method.

Herein, a visually detectable particle refers to a particle that is visually detectable under high illuminance and has a particle size of 40 µm or greater. Among them, particles that are visually detectable under standard illuminance as prescribed in the Japanese pharmacopoeia (about 2,000 to 3,000 lx) are referred to as visible particles (VP) or "insoluble visible particles". Generally, visible particles have a particle size greater than 100 µm (Non Patent Literature 2). Particles that are smaller in size than visible particles, and cannot be seen with eyes with standard illuminance as prescribed in the Japanese pharmacopoeia (about 2,000 to 3,000 lx) but can be visually detected by increasing illuminance or by increasing an observation time period are "particles visually detectable only under high illuminance", and have a particle size of 40 µm to 100 µm. Visible particles can be confirmed by visual inspection with naked eyes for 5 seconds or longer under illumination at standard illuminance (about 2,000 to 3,000 lx), by slowly rotating or inverting the container in front of a black background or a white background. Particles visually detectable only under high illuminance can be confirmed by visual inspection, using a vial visual inspection table, with naked eyes for about 30 seconds under illumination at high illuminance (about 20,000 lx) of a white light source with the container rotated in front of a black background. Visible particles can also be confirmed with inspection under high illuminance. Particles except for those generated from protein molecules in a solution are not considered as "visually detectable particles" regardless of the size. Whether visually detectable particles are derived from protein molecules can be confirmed by Raman microspectroscopic measurement. The only content contained as a protein in the solution is an active pharmaceutical ingredient (API), and visually detectable particles are generated from API. The particle size and number of visually detectable particles can be determined by a light obscuration particle count method, a microscopic particle count method, a flow cytometric particle image analysis method, visual inspection, and infrared microspectroscopy (infrared spectroscopy ;IR) or Raman microspectroscopic measurement performed after isolation of particles, and are measured preferably by a combination of visual inspection and infrared microspectroscopy or Raman microspectroscopic measurement.

In one aspect of the present invention, the term "reducing formation of particles" refers to, in a solution of a pharmaceutical formulation in which visually detectable particles are formed under prescribed conditions, preventing the formation of visually detectable particles or reducing the number of particles to be formed by using a container having a hydrophobic coating on the inside surface. Reduction of formation of visually detectable particles can be confirmed by counting the number of particles obtained under a condition using a container having no hydrophobic coating on the inside surface and the number of particles obtained under a condition using a container having a hydrophobic coating on the inside surface. The size and number of particles can be determined by a light obscuration particle count method, a microscopic particle count method, a flow cytometric particle image analysis method, visual inspection, or infrared microspectroscopy (infrared spectroscopy; IR) or Raman microspectroscopic measurement performed after isolating particles, and are measured preferably by a combination of visual inspection and infrared microspectroscopy or Raman microspectroscopic measurement. In one embodiment of the present invention, reduction of visually detectable particles by the present invention can be confirmed by counting the number of particles under a condition using a container having an inside surface not treated at all (untreated glass vial), and the number of particles under a condition using a container having a hydrophobic coating on the inside surface, and here, an example of the untreated glass vial includes a glass vial neither having a thin film coating including a hydrophobic coating nor having been subjected to additional surface washing with an acid or the like after molding the vial.

In one aspect of the present invention, a pharmaceutical formulation is a solution containing a protein as an active ingredient. The pharmaceutical formulation may be a formulation for injection.

In one aspect of the present invention, a formulation for injection is a pharmaceutical formulation that is filled in a container for injection to be administered by injection, and contains a protein as an active ingredient in a solution.

In one aspect of the present invention, the term "formulation for injection to be obtained" refers to a formulation for injection obtained as a final product.

In one aspect of the present invention, the pharmaceutical formulation is stored without freezing the solution in the container at -30°C to 25°C, preferably from the freezing point of the solution to 25°C, more preferable at 1°C to 10°C, more preferably at 2°C to 8°C, and even more preferably at 5°C. The storage is carried out for 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 24 hours, 25 hours, 26 hours, 27 hours, 28 hours, 29 hours, 30 hours, 31 hours, 32 hours, 33 hours, 34 hours, 35 hours, 36 hours, 48 hours, 60 hours, 72 hours, 84 hours, or 96 hours. The storage is carried out for at least 24 hours, at least 2 days, at least 3 days, at least 4 days, at least 10 days, at least 20 days, at least 30 days, at least 40 days, at least 50 days, at least 60 days, at least 1 month, at least 2 months, at least 3 months, at least 4 months, at least 5 months, at least 6 months, at least 7 months, at least 8 months, at least 9 months, at least 10 months, at least 11 months, or at least 12 months.

In one aspect of the present invention, the protein used in a liquid formulation encompasses, but is not limited to, an antibody, a fusion protein, an enzyme, a hormone, a cytokine, and a vaccine. More specifically, the protein encompasses a monoclonal antibody, granulocyte colony-stimulating factor (G-CSF), granulocyte macrophage colony-stimulating factor (GM-CSF), erythropoietin (EPO), interferon, interleukins such as IL-1 or IL-6, tissue plasminogen activator (TPA), thrombopoietin, urokinase, serum albumin, blood coagulation factor VIII, leptin, stem cell factor (SCF), and the like.

In one aspect of the present invention, the protein used in the pharmaceutical formulation has substantially the same biological activity as a bioactive protein of a mammal, in particular of a human, and encompasses proteins derived from nature and those obtained by genetic engineering. Proteins obtained by genetic engineering include those having the same amino acid sequence as a native protein, or those obtained by deleting, substituting, or adding one or more amino acid sequences and having the above-described biological activity.

In one aspect of the present invention, a concentration of the protein in the solution may be 0.1 mg/mL or more, within a range of 0.1 to 300 mg/mL, or within a range of 1 to 200 mg/mL.

In one aspect of the present invention, an antibody to be used is not particularly limited as long as it binds to a desired antigen, may be a polyclonal antibody or a monoclonal antibody, and is preferably a monoclonal antibody because a homogeneous antibody can be thus stably produced. In one aspect of the present invention, the antibody to be used may be a monospecific antibody or a bispecific antibody, or may be a multispecific antibody having three or more antigen-recognizing sites in a molecule.

In one aspect of the present invention, the protein comprised in the pharmaceutical formulation is an antibody, and in one embodiment, the protein comprised in the pharmaceutical formulation is a monoclonal antibody. In one aspect of the present invention, the protein comprised in the pharmaceutical formulation is an antibody, and after statically storing the solution of the antibody in an untreated glass vial at 40°C for 4 months, one or more visible particles are detected per 1 ml in 60% or more of vials. In one aspect of the present invention, the protein comprised in the pharmaceutical formulation is an antibody, and after statically storing the solution of the antibody in an untreated glass vial at 40°C for 4 months, one or more visible particles are detected per 1 ml in 70% or more of vials. In one aspect of the present invention, the protein comprised in the pharmaceutical formulation is an antibody, and after statically storing the solution of the antibody in an untreated glass vial at 40°C for 4 months, one or more visible particles are detected per 1 ml in 80% or more of vials. Here, an untreated glass vial refers to a glass vial having no coating on the inside surface, and for example, a Schott untreated vial, a Daiwa Special Glass untreated vial, a NIPRO untreated vial, or the like can be used. The solution of the antibody may comprise an additive used in an antibody-containing pharmaceutical formulation, and as the additive, those exemplified herein can be used. Specifically, a buffer, a surfactant, and a stabilizing agent can be used as the additive.

In one aspect of the present invention, the protein comprised in the pharmaceutical formulation is a fusion protein. Herein, a fusion protein refers to a protein including a ligand binding site. Alternatively, it is a polypeptide including a ligand binding molecule further containing a ligand binding domain. The term "ligand binding site" used herein means a portion or a molecule capable of binding to a ligand. In a specific embodiment, examples of the ligand binding site include a ligand receptor, or a portion of a ligand receptor having ligand binding activity.

In one aspect of the present invention, a dose of the protein comprised in the pharmaceutical formulation is 10 µg or greater, 50 µg or greater, 100 µg or greater, 500 µg or greater, 1,000 µg or greater, 5,000 µg or greater, or 10,000 µg or greater. In one embodiment of the present invention, the dose of the protein comprised in the pharmaceutical formulation is 100 µg or greater.

In one aspect of the present invention, a concentration of the protein in the solution is in a range of 0.01 to 1.00 mg/mL, 1.01 to 9.99 mg/mL, 10.0 to 99.9 mg/mL, or 100 to 200 mg/mL. Further, in one aspect of the present invention, the concentration of the protein in the solution is in a range of 1 to 100 mg/mL, 2 to 80 mg/mL, 3 to 60 mg/mL, 4 to 50 mg/mL, 5 to 30 mg/mL, or 6 to 20 mg/mL.

In one aspect of the present invention, the monoclonal antibody to be used encompasses not only a monoclonal antibody derived from an animal, such as a human, a mouse, a rat, a hamster, a rabbit, a sheep, a camel, or a monkey, but also a recombinant antibody obtained by artificial modification, such as a chimeric antibody, a humanized antibody, or a bispecific antibody. Furthermore, a recombinant antibody obtained by artificial modification of a constant region or the like of an antibody for modifying physical properties of an antibody molecule (specifically, modification of an isoelectric point (pI), modification of affinity of Fc receptor, and the like) for purposes of improving retention in blood and pharmacokinetics is also encompassed.

In one aspect of the present invention, the immunoglobulin class of the antibody to be used is not particularly limited, but may be any of classes including IgG such as IgG1, IgG2, IgG3, and IgG4, and IgA, IgD, IgE, and IgM, among which IgG is preferred, and IgG1, IgG2 and IgG4 are particularly preferred.

Further, in one aspect of the present invention, the antibody to be used encompasses not only an antibody including a constant region and a variable region (full-length antibody) but also an antibody fragment, such as Fv, Fab, and F(ab)₂, and a low-molecular-weight antibody like a bispecific antibody such as monovalent or multivalent single chain Fv (scFv, sc(Fv)₂) having a variable region of the antibody bound with a linker such as a peptide linker, or a scFv dimer, and a full-length antibody is preferred. Besides, in one aspect of the present invention, the term "antibody" encompasses a protein fusion antibody obtained by linking a protein to a molecule having an antigen binding site of an antibody (a full-length antibody, an antigen binding fragment of an antibody, or the like), and a peptide fusion antibody obtained by linking a peptide to a molecule having an antigen binding site of an antibody (a full-length antibody, an antigen binding fragment of an antibody, or the like).

In one aspect of the present invention, the antibody to be used can be produced by a known method. A hybridoma used for producing a monoclonal antibody can be produced as follows basically by known techniques. Specifically, a desired antigen or a cell expressing a desired antigen used as a sensitizing antigen is immunized by a usual immunization method, and the resultant immune cell is fused with a known parent cell by a usual cell fusion method, the resultant is subjected to a usual screening method for screening a monoclonal antibody producing cell (hybridoma), and thus, the hybridoma can be produced. The production of a hybridoma can be carried out in accordance with, for example, the method of Milstein et al., (Kohler, G. and Milstein, C., Methods Enzymol.

(1981) 73: 3-46) or the like. If immunogenicity of the antigen is low, the antigen may be bound to a macromolecule having immunogenicity, such as albumin, before the immunization.

Alternatively, a recombinant antibody obtained by cloning an antibody gene from a hybridoma, and incorporating the gene into an appropriate vector to be introduced into a host by genetic engineering techniques can be used (see, for example, Carl, A. K. Borrebaeck, James, W. Larrick, THERAPEUTIC MONOCLONAL ANTIBODIES, Published in the United Kingdom by MACMILLAN PUBLISHERS LTD., 1990). Specifically, cDNA of a variable region (V region) of an antibody is synthesized from mRNA of the hybridoma with reverse transcriptase. DNA encoding the V region of the target antibody thus obtained is linked to DNA encoding a desired antibody constant region (C region), and the resultant is incorporated into an expression vector. Alternatively, DNA encoding the V region of the antibody may be incorporated into an expression vector containing DNA of an antibody C region. The resultant is incorporated into the expression vector so as to express under control of an expression control region, such as an enhancer or a promoter. Next, a host cell is transformed with the resultant expression vector, and thus, the antibody can be expressed.

In one aspect of the present invention, a recombinant antibody obtained by artificial modification for purpose of reducing heteroantigenicity against human, such as a chimeric antibody or a humanized antibody, can be used. Such a modified antibody can be produced by a known method. A chimeric antibody is an antibody containing heavy-chain and light-chain variable regions of an antibody of a mammal other than a human, for example, a mouse antibody, and heavy-chain and light-chain constant regions of a human antibody, and can be obtained by linking DNA encoding a variable region of a mouse antibody to DNA encoding a constant region of a human antibody, incorporating the resultant into an expression vector, and introducing the vector into a host to produce the antibody.

A humanized antibody is designated also as a reshaped human antibody, and is obtained by transplanting a complementary determining region (CDR) of an antibody of a mammal other than a human, for example, a mouse antibody, into a complementary determining region of a human antibody, and a general genetic engineering method for such an antibody is also known. Specifically, a DNA sequence designed to link CDR of a mouse antibody to a framework region (FR) of a human antibody is synthesized by PCR method from several oligonucleotides produced to have overlapping portions at the ends. The thus obtained DNA is linked to DNA encoding a human antibody constant region, the resultant is subsequently incorporated into an expression vector, and the resultant vector is introduced into a host to produce the antibody (see European Patent Application No. 239400, and WO 96/02576). As the FR of a human antibody to be linked via CDR, one having a complementary determining region forming a good antigen-binding site is selected. If necessary, an amino acid in a framework region of a variable region of an antibody may be substituted so as to cause the complementary determining region of the reshaped human antibody to form a suitable antigen-binding site (Sato, K. et al., Cancer Res. (1993) 53, 851-856).

As techniques for substituting an amino acid of an antibody for improving activity, physical properties, pharmacokinetics, safety and the like of the antibody, for example, the following techniques are known, and in one aspect of the present invention, the antibody to be used encompasses such an antibody having substitution (including deletion and addition) of an amino acid.

As techniques for amino acid substitution in a variable region of an IgG antibody, not only humanization (Tsurushita N., Hinton P.R., Kumar S., Design of humanized antibodies: from anti-Tac to Zenapax., Methods, 2005 May; 36 (1): 69-83) but also affinity maturation by amino acid substitution in a complementary determining region (CDR) for enhancing binding activity (Rajpal A., Beyaz N., Haber L., Cappuccilli G., Yee H., Bhatt R.R., Takeuchi T., Lerner R.A., Crea R., A general method for greatly improving the affinity of antibodies by using combinatorial libraries, Proc Natl Acad Sci USA, 2005 Jun 14; 102 (24): 8466-71), and improvement of physicochemical stability by amino acid substitution in framework (FR) (Ewert S., Honegger A., Pluckthun A., Stability improvement of antibodies for extracellular and intracellular applications: CDR grafting to stable frameworks and structure-based framework engineering, Methods, 2004 Oct; 34(2): 184-99. Review) have been reported. As techniques for amino acid substitution in Fc region of an IgG antibody, techniques for enhancing antibody dependent cellular cytotoxicity (ADCC) or complement dependent cellular cytotoxicity (CDC) are known (Kim S. J., Park Y., Hong H. J., Antibody engineering for the development of therapeutic antibodies, Mol Cells, 2005 Aug 31; 20(1): 17-29 Review.). In addition, techniques for amino acid substitution in Fc by not only enhancing such effector function but also improving half-life in blood of an antibody have been reported (Hinton P.R., Xiong J.M., Johlfs M.G., Tang M.T., Keller S., Tsurushita N., An engineered human IgG1 antibody with longer serum half-life, J Immunol. 2006 Jan 1; 176(1): 346-56, Ghetie V., Popov S., Borvak J., Radu C., Matesoi D., Medesan C., Ober R. J., Ward E. S., Increasing the serum persistence of an IgG fragment by random mutagenesis, Nat Biotechnol. 1997 Jul; 15(7): 637-40.). Furthermore, various techniques for amino acid substitution in a constant region for purposes of improving physical properties of an antibody are known (WO 09/41613).

Besides, there are known methods for obtaining a human antibody. For example, human lymphocyte is sensitized with a desired antigen or a cell expressing a desired antigen *in vitro,* and the thus sensitized lymphocyte is fused to a human myeloma cell, for example, U266, and thus, a desired human antibody having a binding activity to an antigen can be obtained (see Japanese Patent Publication No. 1-59878). Furthermore, when a transgenic animal having all repertoires of human antibody genes is immunized with an antigen, a desired human antibody can be obtained (see WO 93/12227, WO 92/03918, WO 94/02602, WO 94/25585, WO 96/34096, and WO 96/33735). Besides, a technique for obtaining a human antibody by panning using a human antibody library is also known. For example, a variable region of a human antibody is expressed as a single chain antibody (scFv) on the surface of a phage by a phage display method, and thus, a phage binding to an antigen can be selected. When the gene of the selected phage is analyzed, a DNA sequence encoding the variable region of a human antibody binding to the antigen can be determined. When the DNA sequence of scFv binding to the antigen is clarified, an appropriate expression vector containing the sequence can be produced to obtain a human antibody. These methods are already known, and can be executed with the reference to WO 92/01047, WO 92/20791, WO 93/06213, WO 93/11236, WO 93/19172, WO 95/01438, and WO 95/15388. In one aspect of the present invention, the antibody to be used encompasses such human antibodies.

When an antibody is produced by isolating an antibody gene once, and introducing the gene into an appropriate host, an appropriate combination of a host and an expression vector can be used. When a eukaryotic cell is used as the host, an animal cell, a plant cell, or a fungal cell can be used. As the animal cell, (1) mammal cells such as CHO, COS, myeloma, BHK (baby hamster kidney), HeLa, and Vero, (2) amphibian cells such as Xenopus oocyte, and (3) insect cells such as sf9, sf21, and Tn5 are known. As the plant cell, a cell derived from the genus *Nicotiana,* such as a cell derived from *Nicotiana tabacum,* is known, and this cell may be callus cultured. As the fungal cell, yeast, for example, the genus *Saccharomyces,* such as *Saccharomyces serevisiae,* and filamentous fungus, for example, the genus *Aspergillus* such as *Aspergillus niger* are known. When a prokaryotic cell is used, there is a production system using a bacterial cell. As the bacterial cell, *Escherichia coli (E. coli)* and *Bacillus subtilis* are known. When a target antibody gene is introduced into such a cell by transformation, and the thus transformed cell is cultured *in vitro,* the antibody can be obtained.

Besides, the antibody to be used in the pharmaceutical formulation encompasses a modified antibody. For example, antibodies binding to various molecules of polyethylene glycol (PEG), cytotoxic drugs and the like can be used (Farmaco. 1999 Aug 30; 54(8): 497-516, Cancer J. 2008 May-June; 14(3): 154-69). Such a modified antibody can be obtained by chemically modifying an antibody. Such a method has been already established in this field.

In one aspect of the present invention, the antibody of the present disclosure may be a chimeric antibody. A chimeric antibody is described in, for example, US Patent No. 4,816,567, and Morrison et al., Proc. Natl. Acad. Sci. USA, 81: 6851-6855 (1984). A chimeric antibody may contain a non-human variable region (variable region derived from, for example, a non-human primate such as a monkey, or a mouse, a rat, a hamster, a rabbit or the like) and a human constant region.

In one aspect of the present invention, the antibody of the present disclosure may be a humanized antibody. Representatively, a humanized antibody is humanized for reducing immunogenicity in a human with specificity and affinity of a parent non-human antibody retained. A humanized antibody representatively contains one or more variable regions, and an HVR, for example, CDR derived from a non-human antibody (or a part thereof) and FR derived from a human antibody sequence (or a part thereof) are present therein. A humanized antibody can optionally contain at least a part of a human constant region. In one embodiment, amino acid residues of FR in a humanized antibody may be substituted with corresponding amino acid residues of a non-human antibody (for example, an antibody from which HVR residues are derived) for, for example, retaining or improving specificity and affinity of the antibody.

A humanized antibody and a method for producing the same are reviewed in, for example, the following (Almagro and Fransson, Front. Biosci. 13: 1619-1633 (2008)), and are described in, for example, the following: Riechmann et al., Nature 332: 323-329 (1988); Queen et al., Proc. Nat'l Acad. Sci. USA 86: 10029-10033 (1989); US Patent Nos. 5,821,337, 7,527,791, 6,982,321, and 7,087,409; Kashmiri et al., Methods 36: 25-34 (2005) (describing specificity determining region (SDR) grafting); Padlan, Mol. Immunol. 28: 489-498 (1991) (describing "resurfacing"); Dall'Acqua et al., Methods 36: 43-60 (2005) (describing "FR shuffling"); and Osbourn et al., Methods 36: 61-68 (2005) and Klimka et al., Br. J. Cancer, 83: 252-260 (2000) (describing "guided selection" approach to FR shuffling).

In one aspect of the present invention, a human framework that may be used in humanization may contain, for example, a framework selected by a "best fit" method (Sims et al., J. Immunol. 151: 2296 (1993)), a framework derived from a consensus sequence of a human antibody belonging to a specific subgroup of a heavy chain or light chain variable region (Carter et al., Proc. Natl. Acad. Sci. USA, 89: 4285 (1992), and Prest et al., J. Immunol., 151: 2623 (1993)), or a framework region derived from screening of FR library (Baca et al., J. Biol. Chem. 272: 10678-10684 (1997) and Rosok et al., J. Biol. Chem. 271: 22611-22618 (1996)).

In one aspect of the present invention, the antibody of the present disclosure may be a human antibody. A human antibody can be produced by various techniques. A human antibody is outlined in, for example, van Dijk and van de Winkel, Curr. Opin. Pharmacol. 5: 368-374 (2001) and Lonberg, Curr. Opin. Immunol. 20: 450-459 (2008). A human antibody may be prepared by administering an immunogen to a transgenic animal having been modified to produce a complete human antibody or a complete antibody containing a human variable region in response to an antigen. Such an animal representatively contains the entire or a part of human immunoglobulin locus, and the entire or a part of the human immunoglobulin locus is present in a state where it is substituted with an endogenous immunoglobulin locus, or it is randomly incorporated outside the chromosome or inside the chromosome of the animal. In such a transgenic mouse, endogenous immunoglobulin locus is usually inactivated. A method for obtaining a human antibody from a transgenic animal is reviewed in Lonberg, Nat. Biotech. 23: 1117-1125 (2005). Besides, make reference to, for example, US Patent Nos. 6,075,181 and 6,150,584 describing XENOMOUSE(TM) technology; US Patent No. 5,770,429 describing HUMAB(R) technology; US Patent No. 7,041,870 describing K-M MOUSE(R); and US2007/0061900 describing VELOCIMOUSE(R) technology. A human variable region from a complete antibody generated from such an animal may be further modified by, for example, combining with a different human constant region.

In another aspect of the present invention, a human antibody can be produced by a method based on a hybridoma. A human myeloma cell and a mouse-human BR>W heteromyeloma cell line for producing a human monoclonal antibody are described in the following (for example, KOZBOR J. IMMUNOL., 133: 3001 (1984); BRODEUR ET AL., MONOCLONAL ANTIBODY PRODUCTION TECHNIQUES AND APPLICATIONS, PP. 51-63 (MARCEL DEKKER, INC., NEW YORK, 1987); and BOERNER ET AL., J. IMMUNOL., 147: 86 (1991)). A human antibody generated through human B cell hybridoma technology is described in LI ET AL., PROC. NATL. ACAD. SCI. USA, 103: 3557-3562 (2006). Other examples of the method include US PATENT No. 7,189,826 (describing production of a monoclonal human IgM antibody from a hybridoma cell line), and NI, XIANDAI MIANYIXUE, 26 (4): 265-268 (2006) (describing a human-human hybridoma). Human hybridoma technology (trioma technology) is described in VOLLMERS AND BRANDLEIN, HISTOLOGY AND HISTOPATHOLOGY, 20(3): 927-937 (2005), and VOLLMERS AND BRANDLEIN, METHODS AND FINDINGS IN EXPERIMENTAL AND CLINICAL PHARMACOLOGY, 27(3): 185-91 (2005).

In another aspect of the present invention, a human antibody may also be generated by isolating an Fv clone variable domain sequence selected from human-derived phage display libraries. Such a variable region sequence can then be combined with a desired human constant region. Techniques for selecting a human antibody from antibody libraries will be described below.

In one aspect of the present invention, the antibody of the present disclosure may be isolated by screening a combinatorial library for an antibody having one or more desired activities. For example, a method for creating a phage display library, a method for screening such a library for an antibody having a desired binding characteristic, and the like are known in this technical field. Such methods are reviewed in Hoogenboom et al. in Methods in Molecular Biology 178:1-37 (O'Brien et al., ed., Human Press, Totowa, NJ, 2001), and are described in, for example, McCafferty et al., Nature 348: 552-554; Clackson et al., Nature 352: 624-628 (1991); Marks et al., J. Mol. Biol. 222: 581-597 (1992); Marks and Bradbury, Molecular Biology 248: 161-175 (Lo, ed., Human Press, Totowa, NJ, 2003); Sidhu et al., J. Mol. Biol. 338(2): 299-310 (2004); Lee et al., J. Mol. Biol. 340(5): 1073-1093 (2004); Fellouse, Proc. Natl. Acad. Sci. USA 101(34): 12467-12472 (2004); and Lee et al., J. Immunol. Methods 284(1-2): 119-132(2004).

In a specific phage display method employed in one aspect of the present invention, repertoires of VH and VL can be separately cloned by polymerase chain reaction (PCR), and recombined randomly in phage libraries, and the phage libraries can then be screened for antigen-binding phage as described in Winter et al., Ann. Rev. Immunol., 12: 433-455 (1994). A phage displays an antibody fragment such as scFv or Fab. Libraries from immunized sources can provide high-affinity antibodies to the immunogen without the requirement of constructing hybridomas. In another embodiment, a naive repertoire can be cloned (for example, from a human) to provide a single source of antibodies to a wide range of non-self or self-antigens without any immunization as described by Griffiths et al., EMBO J, 12: 725-734 (1993). In a still another embodiment, naive libraries can also be created synthetically by cloning unrearranged V-gene segments from stem cells, and using PCR primers containing a random sequence encoding the highly variable region CDR3 and to accomplish rearrangement *in vitro,* as described in Hoogenboom and Winter, J. Mol. Biol., 227: 381-388 (1992). Examples of patent publications describing human antibody phage libraries include US Patent No. 5,750,373, and US Patent Publication Nos. 2005/0079574, 2005/0119455, 2005/0266000, 2007/0117126, 2007/0160598, 2007/0237764, 2007/0292936, and 2009/0002360.

An antibody or an antibody fragment isolated from a human antibody library is herein regarded as a human antibody or a human antibody fragment.

In one aspect of the present invention, the antibody of the present disclosure is a multispecific antibody (such as a bispecific antibody). A multispecific antibody is an antibody having binding specificities in at least two different sites (for example, a monoclonal antibody). In one embodiment, one of the binding specificities is specificity to an antigen, and the other is specificity to another antigen. In another embodiment, a bispecific antibody may bind to two epitopes of different antigens. The bispecific antibody may be used for localizing a cytotoxic agent in a cell expressing the antigen. The bispecific antibody may be prepared as a full-length antibody or an antibody fragment.

A method for producing a multispecific antibody is not limited, and examples include recombinant co-expression of two immunoglobulin heavy chain-light chain pairs having different specificities (for example, Milstein and Cuello, Nature 305: 537 (1983), WO93/08829, and Traunecker et al., EMBO J. 10: 3655 (1991)), and knob-in-hole technology (for example, US Patent No. 5,731,168). A multispecific antibody may be produced by controlling electrostatic steering effects for producing an Fc heterodimer molecule (for example, WO2009/089004 A1); cross-linking two or more antibodies or antibody fragments (for example, US Patent No. 4,676,980 and Brennan et al., Science, 229: 81 (1985)); producing an antibody having two specificities with leucine zipper (for example, Kostelny et al., J. Immunol., 148 (5): 1547-1553 (1992)); producing a bispecific antibody fragment by "diabody" technology (for example, Hollinger et al., Proc. Natl. Acad. Sci. USA, 90: 6444-6448 (1993)); using a scFv dimer (for example, Gruber et al., J. Immunol., 152: 5368 (1994)); or preparing a trispecific antibody (for example, Tutt et al., J. Immunol. 147: 60 (1991)). Alternatively, the multispecific antibody may be an antibody engineered to have three or more functional antigen-binding sites, including an "octopus antibody" (for example, US2006/0025576).

In one aspect of the present invention, the antibody or the antibody fragment thereof of the present disclosure may be a "dual acting Fab" or "DAF" containing one antigen-binding site binding to the antigen and another different antigen (for example, US2008/0069820).

In one aspect, a variant (mutant) of an amino acid sequence of the antibody of the present disclosure can be prepared by introducing an appropriate modification to a nucleic acid encoding a molecule of the antibody, or by synthesizing a peptide. Such a modification may be performed through one of or an appropriate combination of a plurality of deletion, insertion, and substitution of an arbitrary amino acid (residue) in the amino acid sequence. An arbitrary combination of deletion, insertion, and substitution can be employed as long as a final construct possesses a desired characteristic (for example, antigen-binding property).

In one aspect of the present invention, when an antigen variant (mutant) resulting from one of or a plurality of amino acid substitutions is provided, a target site for introducing substitution mutation can contain HVR and FR.

Examples of the antibody used in the pharmaceutical formulation include, but are not limited to, an anti-tissue factor antibody, anti-IL-6 receptor antibody, an anti-IL-6 antibody, an anti-glypican-3 antibody, an anti-CD3 antibody, an anti-CD20 antibody, an anti-GPIIb/IIIa antibody, an anti-TNF antibody, an anti-CD25 antibody, an anti-EGFR antibody, an anti-Her2/neu antibody, an anti-RSV antibody, an anti-CD33 antibody, an anti-CD52 antibody, an anti-IgE antibody, an anti-CD11a antibody, an anti-VEGF antibody, an anti-VLA4 antibody, an anti-HM1.24 antibody, an anti-parathyroid gland hormone-related peptide antibody (anti-PTHrP antibody), an anti-ganglioside GM3 antibody, an anti-TPO receptor agonist antibody, a coagulation factor VIII substitution antibody, an anti-IL31 receptor antibody, an anti-HLA antibody, an anti-AXL antibody, an anti-CXCR4 antibody, an anti-NR10 antibody, and a bispecific antibody of factor IX and factor X.

Examples of a preferable reshaped humanized antibody to be used in the pharmaceutical formulation include a humanized anti-interleukin 6 (IL-6) receptor antibody (tocilizumab, hPM-1, or MRA, see WO92/19759), a humanized anti-HM1.24 antigen monoclonal antibody (see WO98/14580), a humanized anti-parathyroid gland hormone-related peptide antibody (anti-PTHrP antibody) (see WO98/13388), a humanized anti-tissue factor antibody (see WO99/51743), a humanized anti-glypican-3 IgG1κ antibody (codrituzumab, GC33, see WO2006/006693), a humanized anti-NR10 antibody (see WO2009/072604), and a bispecific humanized antibody of factor IX and factor X (ACE910, see WO2012/067176).

In one aspect of the present invention, the pharmaceutical formulation can be prepared, if necessary, by mixing with an appropriate pharmaceutically acceptable medium, or the like into a liquid formulation. The solvent of a liquid formulation is water or a pharmaceutically acceptable organic solvent. Examples of such an organic solvent include propylene glycol (1,2-propanediol), polyethylene glycol 300, polyethylene glycol 400, ethanol, glycerol, and acetic acid. Examples of appropriate pharmaceutically acceptable medium include sterilized water and physiological saline, a stabilizing agent (such as arginine, aspartic acid, methionine, and sucrose), an antioxidant (such as ascorbic acid), a buffer (such as phosphoric acid, citric acid, histidine, or another organic acid), an antiseptic agent, a surfactant (such as PEG, or Tween), a chelating agent (such as EDTA), and a tonicity agent (such as sodium chloride). Other low molecular weight polypeptides, proteins such as serum albumin, gelatin, and immunoglobulin, amino acids such as glycine, glutamine, asparagine, glutamic acid, aspartic acid, methionine, arginine, and lysine, sugars and carbohydrates such as polysaccharides and monosaccharides, and sugar alcohols such as mannitol and sorbitol may be contained. In the case of obtaining a solution for injection, examples include physiological saline, an isotonic solution containing glucose or another auxiliary drug, such as D-sorbitol, D-mannose, D-mannitol, or sodium chloride, and it may be used together with an appropriate solubilizing agent such as alcohol (such as ethanol), polyalcohol (such as propylene glycol, or PEG), and a nonionic surfactant (such as polysorbate 80, polysorbate 20, poloxamer 188, or HCO-50).

In one aspect of the present invention, the buffer to be used in a liquid formulation is prepared using a substance for retaining the pH of the solution. In one aspect of the present invention, a liquid formulation containing high concentration of an antibody has a pH of the solution of preferably 4.5 to 7.5, more preferably 5.0 to 7.0, and even more preferably 5.5 to 6.5. In one aspect of the present invention, a usable buffer can adjust the pH within this range, and is pharmaceutically acceptable. Such a buffer is known to those skilled in the art in the field of liquid formulation, and inorganic salts such as phosphates (sodium or potassium) and sodium bicarbonate; organic salts such as citrates (sodium or potassium), sodium acetate, and sodium succinate; and acids such as phosphoric acid, carbonic acid, citric acid, succinic acid, malic acid, and gluconic acid can be used. Alternatively, Tris buffers, and Good's buffers such as MES, MOPS, and HEPES, histidine (such as histidine hydrochloride), glycine, and such can be used.

Generally, the concentration of the buffer is 1 to 500 mmol/L, preferably 5 to 100 mmol/L and more preferably 10 to 20 mmol/L. In using a histidine buffer, the buffer preferably contains 5 to 25 mmol/L histidine, and more preferably contains 10 to 20 mmol/L histidine.

In one aspect of the present invention, a liquid formulation containing a high concentration of an antibody is preferably stabilized by addition of a stabilizing agent suitable for the antibody corresponding to the active ingredient. In one aspect of the present invention, no significant change is observed in a "stable" liquid formulation containing a high concentration of an antibody for at least 12 months, preferably for 2 years, and more preferably for 3 years at refrigeration temperature (2 to 8°C); or for at least 3 months, preferably for 6 months, and more preferably for 1 year at room temperature (22 to 28°C). For example, a total amount of dimers and decomposed matters after storage at 5°C for 2 years is 5.0% or less, preferably 2% or less, and more preferably 1.5% or less, or the total amount of dimers and decomposed matters after storage at 25°C for 6 months is 5.0% or less, preferably 2% or less, and more preferably 1.5% or less.

In one aspect of the present invention, the solution of the pharmaceutical formulation comprises a protein, a surfactant, a buffer, and a stabilizing agent, each of which may comprise a plurality of components. For example, in one embodiment, the solution comprises arginine and aspartic acid as the stabilizing agent. When a plurality of components is comprised, the concentration of the entire component means a total concentration of the plurality of components.

In one aspect of the present invention, representative examples of the surfactant include nonionic surfactants, for example, sorbitan fatty acid esters such as sorbitan monocaprylate, sorbitan monolaurate, and sorbitan monopalmitate; glycerin fatty acid esters such as glycerin monocaprylate, glycerin monomyristate, and glycerin monostearate; polyglycerin fatty acid esters such as decaglyceryl monostearate, decaglyceryl distearate, and decaglyceryl monolinoleate; polyoxyethylene sorbitan fatty acid esters such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate, and polyoxyethylene sorbitan tristearate; polyoxyethylene sorbit fatty acid esters such as polyoxyethylene sorbit tetrastearate and polyoxyethylene sorbit tetraoleate; polyoxyethylene glycerin fatty acid esters such as polyoxyethylene glyceryl monostearate; polyethylene glycol fatty acid esters such as polyethylene glycol distearate; polyoxyethylene alkyl ethers such as polyoxyethylene lauryl ether; polyoxyethylene polyoxypropylene alkyl ethers such as polyoxyethylene polyoxypropylene glycol ether, polyoxyethylene polyoxypropylene propyl ether, and polyoxyethylene polyoxypropylene cetyl ether; polyoxyethylene alkyl phenyl ethers such as polyoxyethylene nonylphenyl ether; polyoxyethylene hydrogenated castor oils such as polyoxyethylene castor oil, and polyoxyethylene hydrogenated castor oil (polyoxyethylene hydrogen castor oil); polyoxyethylene beeswax derivatives such as polyoxyethylene sorbit beeswax; polyoxyethylene lanolin derivatives such as polyoxyethylene lanolin; polyoxyethylene fatty acid amides such as polyoxyethylene stearic acid amide that have HLB 6 to 18; anionic surfactants, for example, alkyl sulfates having an alkyl group having 10 to 18 carbon atoms such as sodium cetyl sulfate, sodium lauryl sulfate, and sodium oleyl sulfate; polyoxyethylene alkyl ether sulfates such as sodium polyoxyethylene lauryl sulfate having an average added molar number of ethylene oxide of 2 to 4 and an alkyl group having 10 to 18 carbon atoms; alkylsulfosuccinic acid esters having an alkyl group having 8 to 18 carbon atoms such as sodium lauryl sulfosuccinate ester; and natural surfactants, for example, lecithin and glycerophospholipids; sphingo-phospholipids such as sphingomyelin; and sucrose fatty acid esters of fatty acids having 12 to 18 carbon atoms. In one aspect of the present invention, one or more of these surfactants can be added in combination to the formulation.

Preferred surfactants include polyoxyethylene sorbitan fatty acid esters and polyoxyethylene polyoxypropylene alkyl ethers, polysorbates 20, 21, 40, 60, 65, 80, 81, 85, and Pluronic(R) surfactants are particularly preferred, and polysorbates 20 and 80 and Pluronic(R) F-68 (poloxamer 188) are most preferred.

In one aspect of the present invention, an amount of the surfactant to be added to the antibody formulation is generally 0.0001 to 10% (mg/mL), preferably 0.001 to 5%, and more preferably 0.005 to 3%.

To the formulation of the present invention, additives such as a cryoprotectant, a suspending agent, a dissolution assisting agent, a tonicity agent, a preservative, an adsorption inhibitor, a diluent, an excipient, a pH adjustor, a soothing agent, a sulfur-containing reducing agent, and an antioxidant can be appropriately added if necessary.

Examples of the cryoprotectant include sugars such as trehalose, sucrose, and sorbitol.

Examples of the dissolution assisting agent include polyoxyethylene hydrogenated castor oil, polysorbate 80, nicotinamide, polyoxyethylene sorbitan monolaurate, macrogol, and castor oil fatty acid ethyl ester.

Examples of the tonicity agent include sodium chloride, potassium chloride, and calcium chloride.

Examples of the preservative include methyl parahydroxybenzoate, ethyl parahydroxybenzoate, sorbic acid, phenol, cresol, and chlorocresol.

Examples of the adsorption inhibitor include human serum albumin, lecithin, dextran, an ethylene oxide/propylene oxide copolymer, hydroxypropyl cellulose, methyl cellulose, polyoxyethylene hydrogenated castor oil, and polyethylene glycol.

Examples of the sulfur-containing reducing agent include those containing sulfhydryl groups such as N-acetylcysteine, N-acetylhomocysteine, thioctic acid, thiodiglycol, thioethanol amine, thioglycerol, thiosorbitol, thioglycolic acid and salts thereof, sodium thiosulfate, glutathione, and thioalkanoic acids having 1 to 7 carbon atoms.

Examples of the antioxidant include erythorbic acid, dibutylhydroxytoluene, butylhydroxyanisole, α-tocopherol, tocopherol acetate, L-ascorbic acid and salts thereof, L-ascorbic acid palmitate, L-ascorbic acid stearate, sodium hydrogen sulfite, sodium sulfite, triamyl gallate, propyl gallate, and chelating agents such as disodium ethylenediamine tetraacetate (EDTA), sodium pyrophosphate, and sodium metaphosphate.

In one aspect of the present invention, the pharmaceutical formulation is used for treating an autoimmune disease, an immune disease, an infectious disease, an inflammatory disease, a nervous system disease, and a tumor disease and a neoplasm disease including cancer. In a specific embodiment, the pharmaceutical is used for treating congestive heart failure (CHF), ischemia-induced severe arrhythmia, hypercholesteremia, vasculitis, rosacea, acne, eczema, myocarditis, and other states of cardiac muscle, Kawasaki Disease, systemic lupus erythematosus, diabetes, spondylosis, synovial fibroblast, and bone marrow stroma; bone loss; Paget's disease, giant cell tumor of bone; breast cancer; disuse bone loss; malnutrition, periodontal disease, Gaucher's disease, Langerhans cell histiocytosis, spinal cord injury, acute purulent arthritis, osteomalacia, Cushing's syndrome, monostotic fibrous dysplasia, polyostotic fibrous dysplasia, periodontal membrane reconstruction, and fracture; sarcoidosis; melanoma, prostate cancer, pancreas cancer, osteolytic bone cancer, breast cancer, lung cancer, stomach cancer, renal cancer, and rectal cancer; bone metastasis, bone pain management, humoral hypercalcemia of malignancy, ankylosing spondylitis, and other spondylarthrosis; transplant rejection, viral infection, hematological neoplasm, and neoplasm-like state such as Hodgkin's lymphoma; non-Hodgkin's lymphoma (Burkitt's lymphoma, small lymphocytic lymphoma/chronic lymphatic leukemia, mycosis fungoides, mantle cell lymphoma, follicularlymphoma, diffuse large B-cell lymphoma, marginal zone lymphoma, hairy cell leukemia, and lymphoplasmacytic leukemia), tumors of lymphocyte progenitor cells including B-cell acute lymphoblastic leukemia/lymphoma and T-cell acute lymphoblastic leukemia/lymphoma, thymoma, peripheral T-cell leukemia, adult T-cell leukemia/T-cell lymphoma, and tumors of mature T-cell and nature NK cell including large granular lymphocyte leukemia, Langerhans cell histiocytosis, AML with maturation, AML without differentiation, acute myelocytic leukemias including acute promyelocytic leukemia, acute myelomonocytic leukemia, and acute monocytic leukemia, myelodysplastic syndromes, and bone marrow neoplasms such as chronic myeloproliferative disease including chronic myelocytic leukemia, tumors of central nervous system, for example, brain tumor (glioma, neuroblastoma, astrocytoma, medulloblastoma, ependymoma, and retinoblastoma), solid tumor (nasopharyngeal cancer, basal cell carcinoma, pancreatic cancer, cholangiocarcinoma, Kaposi's sarcoma, testicular cancer, uterine cancer, vaginal cancer or cervical cancer, ovarian cancer, primary hepatic cancer, or endometrial cancer, and tumors of vascular system (angiosarcoma and hemangiopericytoma), osteoporosis, hepatitis, HIV, AIDS, spondyloarthritis, rheumatoid arthritis, inflammatory bowel disease (IBD), sepsis and septic shock, Crohn's disease, psoriasis, scleroderma, graft versus host disease (GVHD), allogeneic islet graft rejection, multiple myeloma (MM), myelodysplastic syndromes (MDS), and hematologic malignancy such as acute myelogenous leukemia (AML), inflammation related to tumor, peripheral nerve injury, or demyelinating disease. In a specific embodiment, the pharmaceutical is used for treating psoriasis vulgaris, pancreatitis, ulcerative colitis, non-Hodgkin's lymphoma, breast cancer, colorectal cancer, mesothelioma, soft tissue sarcoma, juvenile idiopathic arthritis, macular degeneration, respiratory syncytial virus, Crohn's disease, rheumatoid arthritis, psoriatic arthritis, Castleman's disease, ankylosing spondylitis, osteoporosis, treatment-induced bone loss, bone metastasis, multiple myeloma, Alzheimer's disease, glaucoma, Sjogren's disease, Still's disease, multiple sclerosis, hyperimmunoglobulinemia, anemia, mesangial proliferative nephritis, and asthma.

In one aspect of the present invention, an antigen to which the antibody has a specific binding property can be a transmembrane molecule (such as a receptor) or a ligand such as a growth factor. Representative examples of the antigen include a molecule such as renin; growth hormones including human growth hormone and bovine growth hormone; a growth hormone-releasing factor; parathyroid gland hormone; thyroid stimulating hormone; lipoprotein; α-1 anti-trypsin; insulin A chain; insulin B chain; proinsulin; follicle-stimulating hormone; calcitonin; luteinizing hormone; glucagon; coagulation factors such as factor VIIIC, factor IX, tissue factor (TF), and von Willebrand factor; anticoagulation factors such as protein C; atrial natriuretic factor; a pulmonary surfactant; plasminogen activators such as urokinase, and human urinary or tissue plasminogen activator (t-PA); bombesin; thrombin; hematopoietic cell growth factor; tumor necrosis factor-α and -β; enkephalinase; RANTES (regulated on activation normally T-cell expressed and secreted); human macrophage inflammatory protein (MIP-1-α); serum albumin such as human serum albumin; Mullerian-inhibiting substance; relaxin A chain; relaxin B chain; prorelaxin; mouse gonadotropin releasing hormone-related peptide; microbial proteins such as β-lactamase; DNAse; IgE; cytotoxic T-lymphocyte related antigens (CTLA) such as CTLA-4; inhibin; activin; vascular endothelial growth factor (VEGF); hormone or growth factor receptors; protein A or D; rheumatoid factor; neurotrophic factor, such as bone-derived neurotrophic factor (BDNF), or neurotrophin-3, -4, -5, or -6 (NT-3, NT-4, NT-5, or NT-6), and nerve growth factor such as NGF-b; platelet-derived growth factor (PDGF); fibroblast growth factors such as aFGF and bFGF; epidermal growth factor (EGF); transforming growth factors (TGF) such as TGF-α, and TGF-β including TGF-b1, TGF-b2, TGF-b3, TGF-b4, and TGF-b5; tumor necrosis factors (TNF) such as TNF-α and TNF-β; insulin-like growth factor-I and -II (IGF-I and IGF-II); des(1-3)-1GF-I (brain IGF-I), and insulin-like growth factor binding protein; CD proteins such as CD3, CD4, CD8, CD19, CD20, CD22, and CD40; erythropoietin; bone morphogenetic protein; an antitoxin; bone morphogenetic protein (BMP); interferons such as interferon-α, -β, and -y; colony stimulating factors (CSF) such as M-CSF, GM-CSF, and G-CSF; interleukins (IL) such as IL-1, IL-2, IL-3, IL-4, IL-5, IL-6, IL-7, IL-8, IL-9, and IL-10; superoxide dismutase; a T-cell receptor; a surface membrane protein; a decay acceleration factor; viral antigens such as a part of AIDS envelope; a transport protein; a homing receptor; addressin; regulatory proteins; integrins such as CD11a, CD11b, CD11c, CD18, ICAM, VLA-4, and VCAM; tumor-related antigens such as HER2, HER3, and HER4 receptors; and a fragment of any of the above-described polypeptides.

In one aspect of the present invention, examples of a molecular target of the antibody comprised therein include CD proteins such as CD3, CD4, CD8, CD19, CD20, CD22, CD34, and CD40; members of the ErbB receptor family such as EGF receptor, and HER2, HER3, or HER4 receptor; B-cell surface antigens such as CD20 and BR3; members of the tumor necrosis receptor superfamily including DR5; prostate stem cell antigen (PSCA); cell adhesion molecules such as LFA-1, Mac1, p150.95, VLA-4, ICAM-1, VCAM, and αv/β3 integrins including any one of α4/β7 integrin, and α or β subunit thereof (such as an anti-CD11a, anti-CD18, or anti-CD11b antibody); growth factors such as VEGF and receptors thereof; tissue factors (TF); tumor necrosis factors (TNF), such as TNF-α or TNF-β, and α-interferon (α-IFN); interleukins such as IL-8; IgE; blood group antigens; flk2/flk3 receptor; obesity (OB) receptor; mp1 receptor; CTLA-4; and protein C.

In one aspect of the present invention, examples of a container to be filled with the pharmaceutical formulation include a vial, a syringe and a cartridge.

In one aspect of the present invention, the inside surface of the glass container has a coating, and at least a part of the inside surface has a water contact angle of 90° or greater, 95° or greater, 100° or greater, or 105° or greater.

Herein, the water contact angle can be measured by a known method. For example, the water contact angle can be measured by a drop method (with a result calculated by a θ/2 method) using a contact angle meter available by purchase, such as a contact angle meter manufactured by Kyowa Interface Science Co., Ltd. (Model No.: DropMaster DM500).

In one aspect of the present invention, a vial is used as the container. In one embodiment, the vial is formed from glass, and a vial satisfying the standards of the Japanese Pharmacopoeia, the United States Pharmacopoeia, the European Pharmacopoeia, or ISO is used. In one embodiment, in facilities for producing and filling a protein-containing solution, the solution is subjected to a sterilization treatment before being filled in a vial. In one embodiment, the vial may be stored, before administering the composition to a patient, for a period of one day, or at least 7 days, or at least 14 days, or at least 1 month, or at least 6 months, or at least 1 year, or at least 2 years. In one embodiment, the vial is exposed to storage and/or transportation conditions.

In one embodiment of the present invention, the container such as a vial is exposed to a mechanical stress. Examples of the mechanical stress include, but are not limited to, drop stress, vibration stress, and rotation stress. In one embodiment of the present invention, the vial is exposed to drop stress once, twice, three times, four times, five times, six times, seven times, eight times, nine times, ten times, eleven times, twelve times, thirteen times, fourteen times, fifteen times, sixteen times, seventeen times, eighteen times, nineteen times, twelve times, twenty one times, twenty two times, twenty three times, twenty four times, twenty five times, twenty five times or more, thirty times or more, or forty times or more. Stress applied to the container at the time of drop is varied not only depending on the number of dropping times but also a drop height, a drop direction and the like. The drop height is, but not limited to, for example, 38.1 cm described in American Society for Testing and Materials (ASTM) D4169. Besides, for purposes of applying equivalent drop stress with high reproducibility, the container may be appropriately packaged so as not to change the direction of the container during the drop. An example of the packaging includes, but is not limited to, "housing a vial in a paper box". Preferably, with respect to the drop height and direction, the container is dropped from the height of 38.1 cm with the surface facing downward in the drop changed in the order of the surface 1, the surface 2, the surface 3 and the surface 4. With this drop defined as one set of the drop, two sets of the drop are performed for applying the drop stress once.

In one aspect of the present invention, the term "pre-filled syringe" means a syringe used as a container and filled with a liquid composition. In one embodiment, a pre-filled syringe contains a pharmaceutical composition for administration to a patient filled in the syringe. Here, the syringe may be covered with a syringe closure, such as, but not limited to, a stopper. In one embodiment, the syringe was filled with the composition in a filling facility for production. The pre-filled syringe includes a syringe for administering a drug. Also, the pre-filled syringe includes a stopper to be inserted into the syringe. The pre-filled syringe further includes a needle to be connected to the syringe, or a leading end portion to be connected to a needle. The pre-filled syringe includes a cap for capping the needle or the leading end portion to be connected to the needle. The drug is a liquid drug, and is, for example, a protein formulation.

The syringe includes a barrel formed in a cylindrical shape having a leading end portion and a base end portion. Also, the syringe is substantially cylindrical. The barrel is a member for containing a drug therein. The barrel of the present embodiment includes, in addition to the leading end portion and the base end portion, a cylindrical portion connecting the leading end portion and the base end portion (see Figure 3). Also, the barrel has a flange portion extending outward (radially outward direction of the cylindrical portion) from the entire outer circumference of the other end in the axial direction of the cylindrical portion. The barrel is formed from a glass material that is transparent and can withstand an internal pressure applied in administering the drug.

On the inner surface of the barrel (for example, the inner surface of the cylindrical portion), silicone oil may be applied as a lubricant for reducing sliding resistance of a piston against the inner surface of the barrel.

In one aspect of the present invention, the silicone oil is polydimethylsiloxane. Some non-restrictive examples of the polydimethylsiloxane include Dow Corning(R) 360 Medical Fluid, non-restrictively including Dow Corning (R) 360 Medical Fluid having a viscosity of 350 centistokes, Dow Corning (R) 360 Medical Fluid having a viscosity of 1,000 centistokes, Dow Corning (R) 360 Medical Fluid having a viscosity of 12,500 centistokes, and Dow Corning (R) MDX4-4159 fluid.

In one aspect of the present invention, the size (standard) of a volume of the syringe is not particularly limited. Specifically, in one aspect of the present invention, the advantageous effect is notable in the case of a small-sized syringe having a volume of 0.5 mL to 5.0 mL, and preferably 1 mL. The volume of a solution contained in a syringe of 1 mL standard is within a range of 0.1 to 1.2 mL, and preferably within a range of 0.2 to 1.1 mL. The volume of the solution contained in a syringe of 2.5 mL standard is within a range of 0.1 to 2.5 mL, and preferably within a range of 0.3 to 2.3 mL. When the pharmaceutical formulation contains an aqueous solution, the volume of the aqueous solution contained in a syringe of 1 mL standard is within a range of 0.1 to 1.2 mL, and preferably within a range of 0.2 to 1.1 mL. The volume of the aqueous solution contained in a syringe of 2.5 mL standard is within a range of 0.1 to 2.5 mL, and preferably within a range of 0.3 to 2.3 mL.

In one aspect of the present invention, the size (standard) of a volume of the cartridge is not particularly limited. Specifically, the volume may be, but is not limited to, 0.5 mL to 20.0 mL, for example, 1.0 mL, 1.5 mL, 1.8 mL, 2.0 mL, 2.2 mL, 3.0 mL, 5.0 mL, 10.0 mL, 15.0 mL, or 20.0 mL.

In one aspect of the present invention, the cartridge to be used may be a standard cartridge for injection made of glass. The dimension and tolerance of a glass injection cartridge are defined in International Standard ISO 13926-1. Stoppers and seals (caps and discs) are described in International Standard ISO 13926-2 and 3. The dimension and tolerance of a ready-to-fill syringe or pre-filled syringe are defined in International Standard ISO 11040-4. In one embodiment, the cartridge to be used may be a cartridge for injection made of glass that meets one or more of the above-discussed International Standards. In another aspect of the present invention, the cartridge to be used may be a cartridge for injection made of glass that does not comply with international standards such as ISO.

The present invention will now be described in more detail with reference to Reference Examples and Examples, and it is noted that the present invention is not limited to these Examples.

### Example 1

Each of solutions containing one of six antibodies (mAb1 to mAb6) (mAb1 to mAb6: 10 mg/mL, buffer: 20 mmol/L histidine, stabilizing agents: 150 mmol/L arginine, and 162 mmol/L aspartic acid, surfactant: 0.5 mg/mL poloxamer 188, pH 6.0) was filtrated with a 0.22 µm filter, and 1.0 mL of the resultant was filled in each of three ISO 2R vials (untreated (NIPRO), Type I plus(R) (SCHOTT), and TopLyo(R) (SCHOTT)) having been subjected to dry-heat sterilization, and the resultant was stoppered with a stopper, and sealed with an aluminum cap. In samples thus filled/stoppered, samples determined to contain insoluble visible particles in the vial by evaluation of insoluble visible particles were excluded, and 20 vials each were tested. Each sample was subjected to, after static storage at 40°C for a prescribed period of time, evaluation of insoluble visible particles and evaluation of insoluble microparticles. In a sample in which the insoluble visible particles were found to be present, the visible particles were identified by Raman spectrum measurement using Raman Imaging Microscope (DXR2xi). The six antibodies used were mAb1 (H-chain: SEQ ID NO: 1, L-chain: SEQ ID NO: 2), mAb2 (anti-CD137 humanized antibody), mAb3 (combination of H-chain: SEQ ID NO: 3 and L-chain: SEQ ID NO: 4, and H-chain: SEQ ID NO: 6 and L-chain: SEQ ID NO: 5; anti-HLA-DQ2.5 humanized bispecific antibody), mAb4 (humanized bispecific antibody having blood coagulation factor VIII (FVIII) cofactor function alternative activity), mAb5 (anti-IL-8 humanized antibody), and mAb6 (humanized multi-specific antibody targeting Claudin-6). The pIs of these were as follows: mAb1: 5.8, mAb2: 9.0, mAb3: 7.3, mAb4: 6.9, mAb5: 9.2, and mAb6: 7.5.

### Evaluation of Insoluble Visible Particles

The outside of each vial was cleaned, and with a vial visual inspection table used, and with the brightness of a white light source in a vial setting position set to a brightness of about 20,000 lx, the vial was rotated to perform visual inspection for about 30 seconds in front of a black background to examine whether or not insoluble visible particles were present in the solution filled in the vial.

### Evaluation 1 of Insoluble Microparticles

The drug solutions filled in three vials having been statically stored at 40°C were put together to be mixed by inverting 20 times, and the resultant mixture was allowed to stand still for 2 hours. Immediately before measurement, the mixture was mixed by slowly inverting 3 times, and the number of microparticles present in the solution was measured with a micro-flow imaging device (Model No.: MFI5200, manufactured by Protein Simple).

### Method for Identifying Insoluble Visible Particles

In each of 3 samples at most out of various vial samples in which insoluble visible particles had been found to be present during the static storage at 40°C by the evaluation of insoluble visible particles, the whole amount of the solution was aspiration filtrated with a nickel filter having a pore size of 3 µm. A Raman spectrum of a foreign matter having the largest size among particles captured on the filter was obtained to perform identification.

### Evaluation Results

The results of the evaluation of insoluble visible particles after the static storage at 40°C are shown in Table 1 below. In all the 6 antibodies (mAb1 to mAb6), insoluble visible particles were found in many samples of the untreated vials and the Type I plus(R) vials. It was confirmed that in the samples stored in the TopLyo(R) vials, the number of insoluble visible particles was remarkably smaller than in the untreated vials and the Type I plus(R) vials. Among the insoluble visible particles visually found in the vials during the static storage at 40°C (for a period of 1 M or 3 M), about 90% of those identified were confirmed to be protein-derived particles. Therefore, it was determined that insoluble visible particles detected by the evaluation of insoluble visible particles after static storage at 40°C for a period of 2 M or 4 M would be mainly protein-derived particles. Accordingly, it was found that the number of protein-derived insoluble visible particles can be reduced by using the TopLyo(R) vial. The results of the evaluation of insoluble microparticles after the static storage at 40°C are shown in Table 2 below. It was confirmed that in the drug solutions stored in the TopLyo(R) vials, the number of insoluble microparticles was smaller than in the drug solutions stored in the untreated vials and the Type I plus(R) vials.

**[Table 1]**

| Table 1 Results of Evaluation of Insoluble Visible Particles after Static Storage at 40°C | | | |
|---|---|---|---|
| Type of Antibody | Type of Vial | Storage Period [month] | Number of Samples Containing Granular Insoluble Visible Particles/Total Number Tested |
| mAb1 | Untreated | 2 | 16/17 |
| | Type I plus^{®} | | 13/17 |
| | TopLyo^{®} | | 1/20 |
| mAb2 | Untreated | | 17/17 |
| | Type I plus^{®} | | 14/17 |
| | TopLyo^{®} | | 3/19 |
| mAb3 | Untreated | | 14/17 |
| | Type I plus^{®} | | 14/17 |
| | TopLyo^{®} | | 0/20 |
| mAb4 | Untreated | 4 | 17/17 |
| | Type I plus^{®} | | 20/20 |
| | TopLyo^{®} | | 4/20 |
| mAb5 | Untreated | | 17/17 |
| | Type I plus^{®} | | 19/20 |
| | TopLyo^{®} | | 0/20 |
| mAb6 | Untreated | | 17/17 |
| | Type I plus^{®} | | 20/20 |
| | TopLyo^{®} | | 0/18 |

**[Table 2]**

| Table 2 Results of Evaluation 1 of Insoluble Microparticles after Static Storage at 40°C | | | | | | |
|---|---|---|---|---|---|---|
| Type of Antibody | Type of Vial | Storage Period [month] | Number of Microparticles( / 1.0 mL) | | | |
| | | | ≥ 2 µm | ≥ 5 µm | ≥ 10 µm | ≥ 25 µm |
| mAbl | Untreated | 2 | 249 | 57 | 23 | 7 |
| | Type I plus^{®} | | 202 | 51 | 13 | 2 |
| | TopLyo^{®} | | 74 | 20 | 8 | 2 |
| mAb2 | Untreated | | 436 | 69 | 13 | 3 |
| | Type I plus^{®} | | 192 | 67 | 23 | 5 |
| | TopLyo^{®} | | 100 | 26 | 7 | 2 |
| mAb3 | Untreated | | 1014 | 192 | 29 | 5 |
| | Type I plus^{®} | | 408 | 113 | 43 | 0 |
| | TopLyo^{®} | | 107 | 20 | 7 | 0 |
| mAb4 | Untreated | 4 | 3433 | 744 | 149 | 16 |
| | Type I plus^{®} | | 3886 | 1011 | 156 | 3 |
| | TopLyo^{®} | | 295 | 43 | 11 | 0 |
| mAb5 | Untreated | | 2356 | 508 | 120 | 8 |
| | Type I plus^{®} | | 1517 | 382 | 90 | 2 |
| | TopLyo^{®} | | 208 | 36 | 7 | 2 |
| mAb6 | Untreated | | 12336 | 3775 | 1192 | 151 |
| | Type I plus^{®} | | 4812 | 1767 | 684 | 115 |
| | TopLyo^{®} | | 238 | 41 | 10 | 0 |

To each sample, a mechanical stress was applied after storage at 40°C for a prescribed period of time, and evaluation of insoluble microparticles was performed.

### Mechanical Stress

Referring to ASTM D4169, stress of a combination of the following drop test and vibration test (in the order of the drop test, the vibration test, and the drop test) was applied.

### - Drop Test

Each vial was housed in a paper box, and protected with a packing material. The paper box was numbered as illustrated in Figure 1 below. With dropping (from a height of 38.1 cm) successively on surfaces 1, 2, 3, and 4 in this order defined as 1 set, each sample packaged in the paper box was subjected to 2 sets of dropping in one drop test.

### - Vibration Test

A vibration stress with Truck Low 40 min, Truck Middle 15 min, Truck High 5 min, and Air level I 120 min was applied to the paper box housing each vial.

### Evaluation 2 of Insoluble Microparticles

In the samples before the application of the mechanical stress and after the application of the mechanical stress, the drug solutions filled in three vials were put together to be mixed by inverting 20 times, and the resultant mixture was allowed to stand still for 2 hours. Immediately before measurement, the mixture was mixed by slowly inverting 3 times, and the number of microparticles present in the solution was measured with a micro-flow imaging device (Model No.: MFI5200, manufactured by Protein Simple).

### Evaluation Results

A difference obtained by subtracting the number of insoluble microparticles obtained before the application of the mechanical stress from the number of insoluble microparticles obtained after the application of the mechanical stress is shown in Table 3 below. When the subtracted result had a value smaller than 0, "0" is shown. In all the six antibodies (mAb1 to mAb6), the number of insoluble microparticles increased by the mechanical stress application was smallest in the drug solutions stored in the TopLyo(R) vials. It was thus found that the number of insoluble microparticles adsorbing to the surface of the vial through the static storage and falling off through the mechanical stress application is remarkably reduced in the TopLyo(R) vials as compared with those in the untreated vials and the Type I plus(R) vials.

**[Table 3]**

| Table 3 Difference in Number of Insoluble Microparticles after Mechanical Stress Application | | | | | | |
|---|---|---|---|---|---|---|
| Type of Antibody | Type of Vial | Storage Period [month] | Number of Microparticles( / 1.0 mL) | | | |
| | | | ≥ 2 µm | ≥ 5 µm | ≥ 10 µm | ≥ 25 µm |
| mAbl | Untreated | 2 | 20253 | 7176 | 2396 | 120 |
| | Type I plus^{®} | | 15892 | 4668 | 1566 | 128 |
| | TopLyo^{®} | | 2665 | 181 | 10 | 0 |
| mAb2 | Untreated | | 35697 | 12458 | 4180 | 269 |
| | Type I plus^{®} | | 24918 | 5362 | 1200 | 31 |
| | TopLyo^{®} | | 955 | 0 | 0 | 0 |
| mAb3 | Untreated | | 57447 | 24889 | 10392 | 1302 |
| | Type I plus^{®} | | 39603 | 13089 | 4120 | 208 |
| | TopLyo^{®} | | 2031 | 184 | 39 | 5 |
| mAb4 | Untreated | 4 | 58049 | 17543 | 4625 | 285 |
| | Type I plus^{®} | | 32739 | 6408 | 1025 | 81 |
| | TopLyo^{®} | | 3368 | 506 | 96 | 10 |
| mAb5 | Untreated | | 20564 | 6160 | 1565 | 92 |
| | Type I plus^{®} | | 15987 | 3845 | 836 | 39 |
| | TopLyo^{®} | | 1136 | 61 | 0 | 0 |
| mAb6 | Untreated | | 32710 | 11486 | 3194 | 123 |
| | Type I plus^{®} | | 22139 | 5539 | 992 | 0 |
| | TopLyo^{®} | | 2351 | 226 | 21 | 0 |

### - Evaluation of Wettability on Container Surface (Measurement of Water Contact Angle)

Each vial was broken to prepare a sample of a vial bottom in a size of about 1 cm in length and width. By employing a drop method using a contact angle meter (Model No.: DropMaster DM500, manufactured by Kyowa Interface Science Co., Ltd.), a water contact angle of each sample 500 milliseconds after dripping was measured. The measurement results were calculated by a θ/2 method.

### Evaluation results

The results of the water contact angles of the bottoms of the respective vials are shown in Table 4.

**[Table 4]**

| Table 4 Measurement Results of Water Contact Angle of Each Vial Bottom (N≥3) | |
|---|---|
| Type of Vial | Contact Angle (°) |
| Untreated | 36.6 |
| Type I plus^{®} | 39.5 |
| TopLyo^{®} | 99.1 |
| Silicon Coat | 106.1 |

### Example 2

Each of solutions containing one of two antibodies (mAb1 and mAb3) (mAb1 and mAb3: 10 mg/mL, buffer: 20 mmol/L histidine, stabilizing agents: 150 mmol/L arginine, and 162 mmol/L aspartic acid, surfactant: 0.5 mg/mL poloxamer 188, pH 6.0) was filtrated with a 0.22 µm filter, and 1.0 mL of the resultant was filled in each of three ISO 2R vials (untreated (NIPRO), TopLyo(R) (SCHOTT), and silicone-coated vial (DAIWA SPECIAL GLASS Co., Ltd.)) having been subjected to dry-heat sterilization, and the resultant was stoppered with a stopper, and sealed with an aluminum cap. In samples thus filled/stoppered, samples determined to contain insoluble visible particles in the vial by evaluation of insoluble visible particles were excluded, and 20 vials each were tested. Each sample was subjected to evaluation of insoluble visible particles after static storage at 40°C for 3 months. In a sample in which the insoluble visible particles were found to be present, the visible particles were identified by Raman spectrum measurement using Raman Imaging Microscope (DXR2xi).

### - Evaluation of Insoluble Visible Particles

The outside of each vial was cleaned, and with a vial visual inspection table used, and with the brightness of a white light source in a vial setting position set to a brightness of about 20,000 lx, the vial was rotated to perform visual inspection for 30 seconds in front of a black background to examine whether or not insoluble visible particles were present in the solution filled in the vial.

### - Method for Identifying Insoluble Visible Particles

In each of 3 samples at most out of various vial samples in which insoluble visible particles had been found by the evaluation of insoluble visible particles, to be present during the static storage at 40°C for 3 months, the whole amount of the solution was aspiration filtrated with a nickel filter having a pore size of 3 µm. A Raman spectrum of a foreign matter having the largest size among particles captured on the filter was obtained to perform identification.

### Evaluation Results

The results of the evaluation of insoluble visible particles after the static storage at 40°C for 3 months are shown in Table 5 below. In the 2 antibodies (mAb1 and mAb3), insoluble visible particles were found in many samples of the untreated vials. It was confirmed that in the samples stored in the TopLyo(R) vials and the silicone-coated vials, the number of insoluble visible particles was remarkably smaller than in the untreated vials. The insoluble visible particles of 2 sample of each of the antibodies and vials in which insoluble visible particles had been visually found after the static storage at 40°C for 3 months, 10 samples in total, were subjected to identification. As a result, it was confirmed, in 8 samples out of the 10 samples, that the particles were protein-derived particles. Therefore, it was determined that the insoluble visible particles detected through the evaluation of insoluble visible particles are mainly protein-derived particles. Accordingly, it was found that the number of protein-derived insoluble visible particles can be reduced by using a silicone-coated vial to the same extent as the TopLyo(R) vial.

**[Table 5]**

| Table 5 Results of Evaluation of Insoluble Visible Particles after Static Storage at 40°C | | | |
|---|---|---|---|
| Type of Antibody | Type of Vial | Storage Period [month] | Number of Samples Containing Granular Insoluble Visible Particles/Total Number Tested |
| mAb1 | Untreated | 3 | |
| | TopLyo(R) | | 0/20 |
| | Silicone Coated | | 2/20 |
| mAb3 | Untreated | | 20/20 |
| | TopLyo(R) | | 7/20 |
| | Silicone Coated | | 5/20 |

## Claims

1. A method for reducing, in a pharmaceutical formulation comprising a protein as an active ingredient in a solution, adsorption of the protein to a container and formation of particles in the solution,
wherein a material of the container is glass, an inside surface of the container has a coating, at least a part of the inside surface has a water contact angle of 90° or greater, and the particles have a particle size of 40 µm or greater.

2. The method according to claim 1, wherein the solution is an aqueous solution.

3. The method according to claim 1 or 2, wherein the protein is a monoclonal antibody, and the monoclonal antibody is any one of a monospecific antibody, a bispecific antibody, and a multi-specific antibody capable of binding to three or more antigens.

4. The method according to any one of claims 1 to 3, wherein the coating is a coating of a hydrophobic compound containing elements Si, C, O, and H.

5. The method according to any one of claims 1 to 4, wherein the water contact angle of the inside surface of the container is 95° or greater.

6. The method according to any one of claims 1 to 5, wherein the container is a vial, a syringe, or a cartridge.

7. The method according to any one of claims 1 to 6, wherein the solution comprises one or a plurality of pharmaceutically acceptable excipients including a buffer, a preservative, an antioxidant, a chelating agent, a cryoprotective agent, a surfactant, a tonicity agent, a stabilizing agent, or a combination thereof.

8. The method according to any one of claims 1 to 7, wherein a pH of the solution is in a range of 4.5 to 7.5.

9. The method according to any one of claims 1 to 8, wherein a dose of the protein is 10 µg or greater, 50 µg or greater, 100 µg or greater, 500 µg or greater, 1,000 µg or greater, 5,000 µg or greater, or 10,000 µg or greater.

10. The method according to any one of claims 1 to 9, wherein a concentration of the protein in the solution is in a range of 0.01 to 1.00 mg/mL, 1.01 to 9.99 mg/mL, 10.0 to 99.9 mg/mL, or 100 to 200 mg/mL.

11. The method according to any one of claims 1 to 10, wherein the protein is a monoclonal antibody selected from an antibody having an H-chain of SEQ ID NO: 1 and an L-chain of SEQ ID NO: 2, and an antibody having an H-chain of SEQ ID NO: 3 and an L-chain of SEQ ID NO: 4, and an H-chain of SEQ ID NO: 6 and an L-chain of SEQ ID NO: 5.

12. The method according to any one of claims 1 to 11, wherein the coating is a coating of a hydrophobic compound containing elements Si, C, O, and H.

13. The method according to claim 12, wherein a content in the hydrophobic compound of other elements except for Si, C, O and H is lower than 10%, and the compound is represented by a composition SiOₓC_{y}H_{z}, wherein x is in a range of 0.0 to 1.2, y is 0.0 to 6.0, and z is in a range of 0.0 to 6.0.

14. The method according to any one of claims 1 to 12, wherein the coating is a coating of polydimethylsiloxane (PDMS).

15. A pharmaceutical formulation comprising a protein as an active ingredient in a solution, wherein a material of a container is glass, an inside surface of the container has a coating, at least a part of the inside surface has a water contact angle of 90° or greater, and particles have a particle size of 40 µm or greater, and
(1) a ratio of visible particles detected in the solution after static storage at 40°C for 2 months after production is 25% or smaller,
(2) a ratio of visible particles detected in the solution after static storage at 40°C for 3 months after production is 35% or smaller, or
(3) a ratio of visible particles detected in the solution after static storage at 40°C for 4 months after production is 45% or smaller.

16. The pharmaceutical formulation according to claim 13, for use in the method according to any one of claims 1 to 14.
